# EUROPEAN PATENT SPECIFICATION

(11) **EP 0 281 162 B1**
(45) Date of publication and mention of the grant of the patent: **12.06.1996**
(21) Application number: 88104568.6
(22) Date of filing: 23.12.1983
(51) Int. Cl.: H01B 3/22, C07C 15/50

(54) **Method for improving the electrical insulating characteristics of a fraction, electrical insulating substance, and electrical appliances containing the same**
Verfahren zum Verbessern der elektrischen Isoliereigenschaften einer Fraktion eines elektrischen Isolierstoffes und diese Stoffe enthaltende elektrische Vorrichtungen
Procédé de modification des caractéristiques électriques d'isolation d'une fraction d'une substance isolante électrique et appareils électriques contenant cette substance

(30) Priority: 25.12.1982 JP 233238/82; 20.07.1983 JP 132331/83
(43) Date of publication of application: 07.09.1988
(62) Divisional of application: 83113081.0
(73) Proprietor: NIPPON PETROCHEMICALS COMPANY, LIMITED, Tokyo (JP)
(72) Inventor: Sato, Atsushi, Tokyo (JP); Endo, Keiji, Yokohama-shi Kanagawa-ken (JP); Kawakami, Shigenobu, Ichikawa-shi Chiba-ken (JP); Yanagishita, Hitoshi, Yokohama-shi Kanagawa-ken (JP); Hayashi, Shozo, Yokohama-shi Kanagawa-ken (JP)
(74) Representative: Strehl Schübel-Hopf Groening & Partner

(56) References cited:
- EP-A- 0 103 868
- FR-A- 2 485 563
- FR-A- 2 514 935
- US-A- 4 111 824
- US-A- 4 365 103

## Description

This invention relates to a method for improving the electrical insulating characteristics of a specific fraction, to an electrical insulating substance and to oil-filled electrical appliances that are filled or impregnated with the same electrical insulating substance. The electrical insulating substance of the invention is quite suitable for use in oil-filled electrical appliances in which insulating materials or dielectric materials made of plastics such as polyolefin are at least partly employed.

### Description of the prior art

Electrical appliances such as oil-filled capacitors, oil-filled power cables and transformers have recently been made to withstand high electric voltages while being small in size. With this tendency, various kinds of plastic films are used together with conventional insulating paper.

There are several electrical insulating oils known that are filled in or impregnated in these electrical appliances. However, the conventional electrical insulating oils such as refined mineral oils, polybutenes, alkylbenzenes and polychlorinated biphenyls have several drawbacks. For example, the use of polychlorinated biphenyls was discontinued because it constitutes a public health hazard. Furthermore, the conventional electrical insulating oils are not satisfactorily compatible with the above-mentioned plastic materials, such as polyolefin films, which are recently being used in oil-filled electrical appliances.

With the requirements of high-voltage durability and size reduction, it is necessary that the electrical insulating oil has a high dielectric breakdown voltage, a low dielectric loss tangent, and good hydrogen gas absorbing capacity.

The hydrogen gas absorbing capacity indicates the stability of the insulating oil against corona discharge (partial discharge) under high electric voltage conditions. The higher the gas-absorbing capacity, the smaller the likelihood of corona discharge, which leads to the advantage of the insulating oil having excellent stability or durability.

In order to meet the requirement of high-voltage use, plastic films such as polyolefin films, polystyrene films and polyester films are used to either partially or completely replace the conventional insulating paper as insulating materials or dielectric materials for electrical appliances such as oil-filled electric cables and capacitors. In view of their dielectric strength, dielectric loss tangent and dielectric constant, polyolefin films, especially polypropylene and cross-linked polyethylene films, are preferred as those insulating or dielectric materials.

When these polyolefin films are impregnated with insulating oils, some oils cause the films to swell to some extent. If a film becomes swollen, the thickness of the insulating layer increases. As a result, the resistance to the flow of insulating oil increases in electrical cables, and insufficient impregnation with insulating oil occurs in electric capacitors, causing the formation of voids (unimpregnated portions) and the undesirable lowering of the corona discharge voltage.

In connection with the above-mentioned conventional electrical insulating oils, the values of the dielectric breakdown voltages (BDV) and the dielectric loss tangents (tan δ) are satisfactory to a certain extent, but the hydrogen gas absorbing capacity or corona discharge characteristics and the stability of the dimensions of polypropylene films are not satisfactory.

The first priority document of the EP 103 868 (published on 28.3.84, prio 16.9.82 and 12.7.83) discloses el. insulating oils comprising diarylalkanes and olefins having two aromatic nuclei. The olefinic component is prepared in a seperate process comprising a dehydration step.

A preparation method for el. insulating oils comprising steps (a) and (b) according to the invention is known (US 4111824). In view of the above-described conventional state of the art, it is the primary object of this invention to provide an improved electrical insulating substance such as electrical insulating oils, and to provide oil-filled electrical appliances that are filled or impregnated with the improved electrical insulating substances.

Another purpose of this invention is to provide an electrical insulating substance that has an excellent dielectric constant and other advantageous properties, a good hydrogen gas absorbing capacity, and high compatibility with insulating or dielectric materials made of plastics.

It is a further object of this invention to provide oil-filled electrical appliances that have excellent corona discharge characteristics, dielectric breakdown voltage and other advantageous electrical characteristics, and to maintain a long service life.

The present invention is therefore directed to a method for improving the electerical insulating characteristics of a fraction which comprises dehydrogenating said fraction at a temperature in the range of 350 to 650°C in the presence of a dehydrogenation catalyst to obtain a dehydrogenated reaction mixture containing at least 0.5% by weight of an aromatic monoolefing or diolefin, said fraction being obtained by the steps of: reacting an aromatic hydrocarbon or hydrocarbons with an alkylating agent in the presence of alkylating catalyst to produce an alkylation product mainly consisting of mono- and polyalkylated aromatic hydrocarbons, diarylalkanes, heavier products and unreacted substances, then separating unreacted substances and mono- and polyalkylated aromatic hydrocarbons to obtain a separated fraction; and if desired, refining said separated fraction.

The invention further relates to the improved electrical insulating substance obtained by the above-stated method.

Subject matter of the present invention is furthermore an electrical appliance which is impregnated or filled with the improved electrical insulating substance obtained by the above-defined method.

In the present invention, an aromatic hydrocarbon having two condensed or noncondensed aromatic nuclei is used as a raw material. The aromatic hydrocarbon can be any compound in which at least one olefinic double bond is produced by dehydrogenation. In other words, the aromatic hydrocarbon is a compound that has two condensed or noncondensed aromatic nuclei and at least one aliphatic or alicyclic hydrocarbon residual group having two or more carbon atoms. These hydrocarbon residual groups are mono-valent or poly-valent hydrocarbon groups that are derived from alkanes such as ethane, propane, butane, pentane, hexane, heptane and octane, and cycloalkanes such as cyclopentane and cyclohexane. However, it should be noted that the aromatic hydrocarbon of about 500 or less in molecular weight is generally preferable because the dehydrogenation is easy and the electrical properties of obtained product are good.

The aromatic hydrocarbons are exemplified by diarylalkane, diarylcycloalkane, arylindane, alkylbiphenyl, cycloalkylbiphenyl, alkylnaphthalene and cycloalkylnaphthalene.

The diarylalkane and diarylcycloalkane are exemplified by 1,1-diarylalkanes such as 1,1-diphenylethane, 1-phenyl-1-tolylethane, 1-phenyl-1-xylylethane and 1-phenyl-1-ethylphenylethane; 1,2-diarylalkanes such as 1-phenyl-2-ethylphenylethane and 1-phenyl-2-isopropylphenylethane; and diphenylcyclohexane.

The alkylbiphenyl and cycloalkylbiphenyl are exemplified by monoisopropylbiphenyl, diisopropylbiphenyl and cyclohexylbiphenyl.

The alkylnaphthalene and cycloalkylnaphthalene are exemplified by ethylnaphthalene, isopropylnaphthalene and diisopropylnaphthalene.

As a raw material for preparing the electrical insulating substance of this invention, there is used a fraction that is obtained in the alkylation process to prepare alkylated aromatic hydrocarbons. More particularly, the fraction is obtained by reacting aromatic hydrocarbons with an alkylating agent in the presence of an alkylation catalyst to obtain an alkylation product containing mono- and polyalkylated aromatic hydrocarbons,diarylalkanes, heavier products, and unreacted materials, and then separating mono- and polyalkylated monoaromatic hydrocarbons and unreacted materials from the above-obtained alkylation product.

For the above alkylation, various aromatic hydrocarbons and alkylating agents can be used. For example, if raw materials produce alkylated aromatic hydrocarbons, diarylalkanes and heavier products such as polyaromatic hydrocarbons, and the polyaromatic hydrocarbons can be converted into olefins such as monoolefins and diolefins by dehydrogenation, such material can also be used.

Accordingly, exemplified as these raw materials are benzene; alkylbenzenes such as toluene and xylene; partially hydrogenated condensed polyaromatic hydrocarbon such as indane; and condensed polyaromatic hydrocarbons such as naphthalene and indene.

The alkylating agents are exemplified by ethylene, propylene and butene; alkanols such as ethanol, propanol and butanol; and halogenated paraffin such as ethyl chloride.

In the alkylation reaction, some of the above aromatic hydrocarbons and alkylating agents are selected and used. In this reaction, conventional alkylation catalysts can be used. For example, there are Friedel-Crafts catalysts such as aluminum chloride, mineral acid such as sulfuric acid, organic acid such as p-toluenesulfonic acid, super acid such as trifluoromethane sulfonic acid, and solid acids such as silica alumina and zeolite.

After the alkylation, unreacted material such as benzene, and mono-alkylated and polyalkylated monoaromatic hydrocarbons are separated from the alkylation product and dehydrogenation is then carried out.

In view of the facts that the process is carried out in an industrial scale, those by-products are also obtained in a large quantity and at low cost, and those properties of its dehyrogenation product are better as electrical insulating substances, the following alkylation is a process to obtain a preferable fraction. In order to prepare polystyrene or polyvinyltoluene, benzene or toluene is reacted with ethylene in the presence of an alkylation catalyst to obtain alkylation products containing unreacted benzene, ethylbenzene, polyethylbenzene, 1,1-diphenylethane as a diarylalkane, and heavier product.

Accordingly, the present invention will be further described in more detail with reference to this alkylation.

The alkylation of benzene or toluene with ethylene can be done by known methods, for example, the liquid phase alkylation method or the gas phase alkylation method.

The molar ratio of benzene to ethylene is in the range of, for instance, from about 25:1 to 2:1, preferably from about 10:1 to 3:1. As the alkylation catalysts, solid acid catalysts, mineral acids, and Friedel-Crafts catalysts are preferably used, these catalysts are exemplified by alumina, silica alumina, zeolite, sulfuric acid, hydrofluoric acid, phosphoric acid, sulfonic acid, p-toluene sulfonic acid, aluminum halides such as aluminum chloride and aluminum bromide, zinc chloride, iron chloride and boron fluoride.

In the liquid phase reaction, the reaction temperature is in the range of about 20 to 175°C, preferably about 90 to 150°C. The reaction pressure is any value that is sufficient to maintain a reaction mixture in a liquid state, for example, from about 0.5 to 14 kg/cm². The reaction time is generally in the range of about 10 minutes to 10 hours, preferably 20 minutes to 3 hours.

In the gas phase reaction, available catalysts are phosphoric acid carried on diatomaceous earth, silica, alumina, or aluminum silicate, and solid acid catalysts such as alumina carried on silica gel. The reaction temperature is in the range of about 250 to 450°C, preferably about 300 to 400°C, and the reaction pressure is in the range of about 25 to 85 kg/cm², and preferably about 42 to 70 kg/cm².

As industrial methods for preparing ethylbenzene by reacting ethylene with benzene, there is a known aluminum chloride method using aluminum chloride as a catalyst, a high pressure method using an alumina catalyst carried on silica gel developed by Koppers Company Inc., a solid phosphoric acid method using a solid catalyst prepared by impregnating diatomaceous earth with phosphoric acid developed by Universal Oil Products Co., the Alkar method using a catalyst of boron fluoride or complex, also developed by Universal Oil Products Co., and the method developed by Mobil Oil Co. using a crystalline zeolite catalyst such as ZSM-5 (trade mark).

Through the foregoing reaction, an alkylation product containing unreacted materials such as benzene and ethylene, mono- and polyethylbenzene, 1,1-diphenylethane, 1-phenyl-1-ethylphenylethane, 1,1-di(ethylphenyl)ethane and heavier products, can be obtained.

This alkylation product is then subjected to usual steps of settling and filtration to remove any alkylation catalyst such as an aluminum chloride catalyst. After that the filtrate is rinsed with water and neutralized.

In the next step, the unreacted benzene and monoaromatic hydrocarbons such as ethylbenzene and polyethylbenzene are removed from the alkylation product through a conventional method such as distillation. The thus obtained residual fraction contains an asphaltic substance that is so far used only as fuel or the like. This asphaltic substance is then removed by a separation process as distillation, as a distillation bottom to obtain a fraction that is to be subjected to dehydrogenation in accordance with this invention.

In the case of alkylation of benzene or toluene with ethylene, the above fraction has boiling points in the range of about 255 to 420°C, preferably about 260 to 400°C, and more preferably about 268 to 400°C. The substances contained in the fraction of this boiling range are 1,1-diphenylethane, 1-phenyl-1-ethylphenylethane, 1,1-di(ethylphenyl)ethane, and heavier products.

The refining treatment, such as neutralization, to remove the residue of the alkylation catalyst can be carried out before or after the dehydrogenation. This refining treatment is done by using an appropriate organic or inorganic basic substance. As the basic substances, there are alkali metals of group I and alkaline earth metals of group II of the periodic table, their oxides and the hydroxides. More particularly, lithium, sodium, potassium, magnesium, calcium, strontium and barium, their oxides and their hydroxides are preferable. The refining treatment can be performed in ordinary manner, and also it is possible to use the above metallic oxide in the distillation step. Further, the refining treatment can be carried out by using absorbents such as clay, silica, alumina and silica·alumina.

The above-described starting materials are dehydrogenated, and the obtained dehydrogenation product or dehydrogenated reaction mixture is employed in this invention.

The dehydrogenation is carried out in the presence of a catalyst, in which any known dehydrogenation catalyst can be used. For instance, exemplified as the catalysts are oxides of metals such as chromium, iron, copper, potassium, magnesium and calcium, precious metals such as platinum and palladium, or those metal oxides or precious metals that are supported on a carrier of alumina or the like.

The reaction temperature of dehydrogenation is in the range of 350 to 650°C, preferably 400 to 600°C. When the dehydrogenation is carried out through a continuous fixed bed process, the LHSV (liquid hourly space velocity) is in the range of 0.2 to 10, preferably 0.5 to 3.0.

In the dehydrogenation, an inert gas such as steam can be introduced into the reaction system. Further, if necessary, a suitable diluent can be used. However, when the rate of dehydrogenation is not so high, raw materials themselves conveniently serve as a diluent.

In the dehydrogenation process of this invention, the above catalysts and reaction conditions are properly selected in order to suppress the occurrence of side effects such as craking (decomposition) or polymerization. However, it is necessary that the dehydrogenation must be so carried out as to produce more than 0.5 wt%, preferably more than 5.0 wt% in the dehydrogenated reaction mixture, of the aromatic monoolefin or diolefin having two condensed or noncondensed aromatic nuclei. When the content of the above aromatic olefin is less than 0.5 wt%, this invention cannot be expected to be effective.

Incidentally, complete dehydrogenation is generally difficult and it is accompanied by decomposition and polymerization at higher conversion rates. Heavier components produced by polymerization and lighter aliphatic or cycloaliphatic components produced by decomposition are not of use, however, the aromatics having two aromatic nuclei sometimes produced by partial decomposition, are rather desirable components in the present invention, because the synergistic effect among produced olefins and unreacted starting material and/or the above partially decomposed aromatics can be expected.

Therefore, after the reaction, hydrogen, and if necessary, lighter components such as aliphatic or cycloaliphatic hydrocarbons produced by side reaction such as decomposition and heavier components produced by polymerization are removed by distillation, thereby obtaining the dehydrogenated reaction mixture, that is, the electrical insulating substance of this invention.

From the above-described electrical insulating substance, an electrical insulating oil can be prepared by the following procedure:

If the obtained reaction mixture has a proper viscosity and the content of the above-mentioned aromatic olefins in the mixture is appropriate, the dehydrogenated reaction mixture can be used as it stands as an electrical insulating oil. When the viscosity is too high or the content of the aromatic olefins is too large, the dehydrogenated reaction mixture is mixed and dissolved together with a proper quantity of conventional electrical insulating oil or oils to prepare easily an excellent electrical insulating oil Furthermore, when the above electrical insulating substance is a solid material at ordinary temperatures but melts at elevated temperatures of usual impregnation, the electrical insulating substance can be used by heating and fusing it for impregnation. In another manner of use, the electrical insulating substance is dissolved into any suitable electrical insulating oil having dissolving power, thereby obtaining an excellent electrical insulating substance in accordance with this invention.

Incidentally, when the above-mentioned aromatic olefins prepared by dehydrogenation are used, they can be isolated through a separating process such as distillation, extraction or recrystallization. The boiling points of components are generally close to each other and any undesirable effect such as polymerization of the aromatic olefins is hardly observed. In addition, the electrical appliances made by using the dehydrogenation product show quite excellent electrical performance because of a synergistic effect. Accordingly, the electrical insulating oil is preferably prepared by using the electrical insulating substance of this invention without applying the above isolation process of the aromatic olefins. Therefore, it is preferable to use the dehydrogenated reaction mixture alone or the combination of the mixture with other electrical insulating oils.

As long as the electrical insulating substance of the invention can be dissolved, any quantity of the following conventional electrical insulating oils can be mixed together. Such insulating oils are exemplified by mineral oils, olefin oligomers such as polybutene, alkyl-or cycloalkylbenzenes such as dodecylbenzene and dicyclohexylbenzene, animal and vegetable oils such as castor oil as a triglyceride, phthalic esters such as dioctylphthalate, and silicone oil.

In addition to the above electrical insulating oils, saturated compounds having two condensed or noncondensed aromatic nuclei and aromatic olefins having at least two condensed or noncondensed aromatic nuclei can be used by mixing them with the electrical insulating substance of this invention.

The saturated compounds having two condensed or noncondensed aromatic nuclei are exemplified by diphenylmethane; lower alkyl derivatives of diphenylmethane such as benzyltoluene; 1,1-diphenylethane, 1,2-diphenylethane or their lower alkyl derivatives such as 1-phenyl-1-xyxlylethane, 1-phenyl-1-ethylphenylethane, 1-phenyl-1-tolylethane, 1-phenyl-1-isopropylphenylethane, 1-phenyl-1-trimethylphenylethane, 1,1-di(ethylphenyl)ethane, 1-phenyl-2-ethylphenylethane, 1-phenyl-2-isopropylphenylethane, and 1,2-dixylylethane; diarylalkanes and diarylcycloalkanes such as 1,3-diphenyl-butane, 2,4-diphenyl-2-methylpentane and diphenylcyclohexane; alkylarylindane such as methylphenylindane; biphenyl; alkyl- or cycloalkylbiphenyls such as mono- or diisopropylbiphenyl and cyclohexylbiphenyl; alkylnaphthalenes such as mono- or diisopropylnaphthalene; ethers such as ditolyl ether, dixylyl ether, dibenzyl ether, bis (α-methylbenzyl)ether and diaryl thioether; and triarylalkanes such as dibenzyltoluene, distyrenated xyxlene, bisphenetyltoluene, terphenyl, styrenated naphthalene, and triphenylhexane.

The above-mentioned aromatic olefins having at least two condensed or noncondensed aromatic nuclei are exemplified by 1,1-diphenylethylene; stilbene; unsaturated dimers or trimers of styrenes including styrene, vinyltoluene, α-methylstyrene and isopropenyltoluene, such as 1,3-diphenylbutene-1, and 2,4-diphenyl-4-methylpentene-1; vinylphenyl-phenylalkanes and their lower alkyl derivatives such as 1-vinylphenyl-1-phenylethane, 1-isopropenylphenyl-1-phenylethane and vinylphenyl-phenylmethane; and other aromatic monoolefins such as isopropenylbiphenyl and isopropenylnaphthalene.

One or a mixture of two or more of the above known insulating oils can be used in combination with the dehydrogenation product of this invention.

In order to improve further the oxidation stability, several known antioxidants can be added to the electrical insulating oil of this invention. As such antioxidants, there are phenol compounds like 2,6-di-tert-butyl-p-cresol, 2,2ʹ-methylenebis(4-methyl-6-tert-butylphenol), 4,4ʹ-butylidenebis(3-methyl-6-tert-butylphenol), 4,4ʹ-thiobis(3-methyl-6-tert-butylphenol), stearyl-β-(3,5-di-tert-butyl-4-hydroxyphenol)propionate, tetrakis[methylene-3(3ʹ,5ʹ-di-tert-butyl-4ʹ-hydroxyphenyl)-propionate]methane, 1,3,5-trimethyl-2,4,6-tris(3,5-di-tert-butyl-4-hydroxybenzyl)benzene, and 1,1,3-tris(2-methyl-4-hydroxy-5-tert-butylphenol)butane; sulfur compounds such as dilauryl thiodipropionate, distearyl thiodipropionate, laurylstearyl thiodipropionate, and dimyristyl thiodipropionate; and phosphorous compounds such as triisodecylphosphite, diphenylisodecylphosphite, triphenylphosphite, and trinonylphenylphosphite.

These antioxidants can be added to the electrical insulating substance singly or in combination of two kinds or more. The additional quantity of the antioxidant is 0.001 to 5 % by weight and preferably 0.01 to 2.0 % by weight of the electrical insulating substance.

Furthermore, in order to impart a nonflammable property and other desirable effects to the electrical insulating substance of this invention, several known additives such as phosphoric esters and epoxy compounds can be added to the electrical insulating substance.

When the electrical insulating substance of this invention is used as an electrical insulating oil, it shows excellent compatibility with insulating materials made of plastics such as polyolefins. Accordingly, when the electrical insulating substance is used for impregnating oil-filled electrical appliances having dielectric or insulating materials partially or totally made of plastics, high-tension, light-weight and long-life electrical appliances can be provided. Furthermore, the electrical insulating substance of this invention can be mixed with or dissolved in known electrical insulating oils including hydrocarbons having at least two condensed or noncondensed aromatic nuclei such as diarylalkanes, alkylbiphenyls and alkylnaphthalenes, and as a result, similar advantages can be attained with the synergistic effect of combined components.

Accordingly, the electrical insulating substance of this invention, or the electrical insulating oil containing the same, is suitable for use as an electrical insulating oil for general uses and is especially suitable for use in impregnation of oil-filled electrical appliances such as capacitors, oil-filled power cables and transformers.

As described at the beginning of this specification, the requirements of high-voltage withstanding and size reduction of such oil-filled electrical appliances have become severe in recent years. In order to meet these requirements, plastics are used to replace either partially or totally the conventional insulating paper as insulating materials or dielectric materials for the oil-filled electrical appliances. More particularly, as electrical insulating materials (dielectric materials) of electric capacitors, there is proposed the use of a combination of insulating paper and plastic films. As the plastic films, stretched or nonstretched polypropylene, polymethylpentene, polyester, polyvinylidene fluoride and polycarbonate films can be used. The use of these plastic films singly, the use of the embossed films of these plastic films to facilitate impregnation with the insulating oil, or the use of single-side or double-side metallized plastic films, where the metallic layer serves as an electrode, is also possible. In the case of oil-filled cables, the electrical insulating materials are made of polyolefin films such as cross-linked or non-cross-linked polyethylene film, stretched or nonstretched polypropylene film, and polymethylpentene film; paper-polyolefin laminated film made by the extrusion of polyolefin onto paper; composite film that is made by cross-linking insulating paper with silane-grafted polyethylene in the presence of a silanol condensation catalyst; or an artificial paper sheet that is made by mixing wood pulp and polyolefin fiber.

When an electric capacitor is provided with an insulating (dielectric)material that is partially or totally made of plastics, especially polyolefin, and when it is impregnated with the electrical insulating oil (including the electrical insulating substance itself) of the present invention, the insulating material can be fully and completely impregnated with the electrical insulating oil. This is because swelling of the insulating material is slight, and voids (unimpregnated portions) are not formed. Accordingly, corona discharge due to the convergence of electric fields to the voids hardly occurs, and dielectric breakdown can be well avoided. Furthermore, the electrical insulating oil of this invention has excellent hydrogen gas absorbing capacity and corona discharge resistance under high-voltage stress, so that it is possible to obtain both a long service life and high-voltage use of the electrical appliances.

In the case of electric power cables, a change in dimensions of the insulating material due to swelling is small, and resistance to the insulating oil flow can be made low so that oil impregnation can be performed in a short time. Of course, it will be understood that, because of the ease of impregnation, voids are hardly formed and the dielectric breakdown voltage becomes higher. When a cable is made by using an insulating material of a laminated film or composite film made of plastic material and paper, peeling, creasing and buckling of the insulating material upon bending of the cable do not occur even when the insulating material has been in contact with the electrical insulating oil for a long time. Further, as in the case of the electric capacitor, a power cable having a good corona discharge resistance can be obtained due to the excellent hydrogen gas absorbing capacity of the electrical insulating oil. Accordingly, it is also possible to obtain a long service life and high-voltage use, as for the capacitors.

According to this invention, the above-described advantageous features can be improved by impregnation with the electrical insulating oil consisting of a plurality of specific component materials, owing to the synergistic effect between the component materials. Further, the good electrical characteristics, biodegradability, thermal resistance, and oxidation stability of each component material can be well maintained, and at the same time, the viscosity and pour point of the electrical insulating oil composition can be adjusted within desired ranges. Therefore, the manufacture of oil-filled electrical appliances is facilited, and oil-filled electrical appliances exhibiting high performance under any use conditions can be obtained.

In the following, the electrical insulating oil and electrical appliances impregnated therewith according to this invention will be described in more detail with reference to the example.

### EXAMPLE 1

In a process for preparing ethylbenzene, which is used for production of polystyrene, benzene was reacted with ethylene in the presence of an aluminum chloride catalyst. From the reaction mixture in this process, unreacted benzene, ethylbenzene and polyethylbenzene were distilled off by reduced pressure distillation to obtain a fraction having a boiling range of 260 to 310°C (normal pressure).

The main components in this fraction were 37 wt% of 1,1-diphenylethane and 32 wt% of 1-phenyl-1-ethylphenylethane. Besides them, 1,1-di(ethylphenyl)ethane, tetralin, indane, naphthalene, fluorene, their alkyl derivatives, and unknown substances were also contained in the fraction.

The recovered fraction was dehydrogenated in the presence of a catalyst and steam under the following conditions.
- Catalyst:: Iron oxide catalyst containing promotors of potassium carbonate and chromium oxide Trade mark: G64A, made by Nissan Girdler Catalyst Co., Ltd.
- Temperature:: 500°C
- LHSV:: 1.0
- H₂O/Starting Material:: 3.0 (molar ratio)
- Pressure:: Atmospheric pressure

After the dehydrogenation, lighter components and heavier components were removed to obtain a dehydrogenation product. The bromine number of this dehydrogenation product was 15.3 cg/g. Assuming that contained olefins are all monoolefins, this value corresponds to about 19 wt% in olefin content. This dehydrogenation product was used as an electrical insulating oil for the following capacitor tests.

The dehydrogenated product and the foregoing recovered fraction before dehydrogenation were used as electrical insulating oils and capacitors were impregnated with these oils. The performances of the capacitors were evaluated by corona starting voltages (CSV), corona ending voltages (CEV) and breakdown times.

The capacitors were made by winding two-ply capacitor-use polypropylene film (each 14 µ thickness) as dielectric material and aluminum foil as electrodes. The capacitance of the obtained capacitors after impregnation was 0.4 µF. The breakdown times were represented by values relative to those of the recovered fraction before dehydrogenation. In the capacitor tests, 0.2 wt% of BHT as an antioxidant was added to both the oils. The results are shown in the following Table 1.

**Table 1**

| Impregnation Oil | CSV (kV) | CEV (kV) | Breakdown Time (Relative Value) |
|---|---|---|---|
| Recovered Fraction before Dehydrogenation | 2.6 | 2.0 | 1 |
| Dehydrogenation Product | 3.1 | 2.5 | 16.3 |

### ELECTRICAL CHARACTERISTICS TEST

With regard to the electrical insulating oils of this invention that were used for the impregnation in the foregoing Example, some electrical characteristics as insulating oils were measured. The results are shown in Table 2.

**Table 2**

| | Electrical Characteristics | | |
|---|---|---|---|
| Example | ε at 80°C | tan δ %, at 80°C | ρ Ω·cm, at 80°C |
| 1 | 2.49 | 0.023 | 9.0x10¹⁴ |

## Claims

1. A method for improving the electrical insulating characteristics of an oil fraction, which comprises dehydrogenating said fraction at a temperature in the range of 350 to 650°C in the presence of a dehydrogenation catalyst to obtain a dehydrogenated reaction mixture containing at least 0.5 percent by weight of an aromatic monoolefin or diolefin having two condensed or non-condensed aromatic nuclei, said fraction to be improved being obtained by the following process steps:
(a) reacting one or more aromatic hydrocarbons with an alkylating agent in the presence of an alkylating catalyst to produce an alkylation product mainly consisting of mono- and polyalkylated aromatic hydrocarbons, diarylalkanes, heavier products and unreacted substances,
(b) separating unreacted material, monoaromatic hydrocarbons and asphaltic. substances from the alkylation product to obtain an alkylation product fraction having boiling points in the range of 255 to 420°C, and
(c) if desired refining said alkylation product fraction.

2. The method according to claim 1, wherein
said alkylation product is obtained by reacting benzene or toluene with ethylene, and mainly contains benzene, ethylbenzene, polyethylbenzene, 1,1-diphenylethane and heavier reaction products.

3. The method according to claim 2, wherein
said alkylation product is obtaind by reacting benzene with ethylene at temperatures in the range of about 20 to 175°C in the presence of an aluminum chloride catalyst.

4. The method according to claim 2, wherein
said reaction of benzene with ethylene is carried out in the presence of a zeolite catalyst.

5. Insulating substance having improved electrical insulating characteristics, obtained by the process according to any of the claims 1 to 4.

## Patentansprüche

1. Verfahren zur Verbesserung der elektrischen Isoliereigenschaften einer Ölfraktion, das die Dehydrierung der Fraktion bei einer Temperatur im Bereich von 350 bis 650 °C in Gegenwart eines Dehydrierkatalysators umfaßt, wobei ein dehydriertes Reaktionsgemisch, das mindestens 0,5 Gew.-% eines aromatischen Monoolefins oder Diolefins mit zwei kondensierten oder nicht-kondensierten aromatischen Kernen enthält, erhalten wird, wobei die zu verbessernde Fraktion durch folgende Verfahrensstufen erhalten wird:
(a) Umsetzung eines oder mehrerer aromatischer Kohlenwasserstoffe mit einem Alkylierungsmittel in Gegenwart eines Alkylierungskatalysators, wobei ein Alkylierungsprodukt hergestellt wird, das hauptsächlich aus mono- und polyalkylierten aromatischen Kohlenwasserstoffen, Diarylalkanen, schwereren Produkten und nichtumgesetzten Substanzen besteht,
(b) Abtrennung der nichtumgesetzten Substanzen, der monoaromatischen Kohlenwasserstoffe und asphaltartigen Substanzen aus dem Alkylierungsprodukt, wobei eine Fraktion des Alkylierungsprodukts mit Siedepunkten im Bereich von 255 bis 420 °C erhalten wird, und
(c) falls erwünscht, Raffination der Fraktion des Alkylierungsprodukts.

2. Verfahren gemäß Anspruch 1, wobei das Alkylierungsprodukt durch Umsetzung von Benzol oder Toluol mit Ethylen erhalten wird und hauptsächlich Benzol, Ethylbenzol, Polyethylbenzol, 1,1-Diphenylethan und schwerere Reaktionsprodukte enthält.

3. Verfahren gemäß Anspruch 2, wobei das Alkylierungsprodukt durch Umsetzung von Benzol mit Ethylen bei einer Temperatur im Bereich von ungefähr 20 bis 175 °C in Gegenwart eines Aluminiumchlorid-Katalysators erhalten wird .

4. Verfahren gemäß Anspruch 2, wobei die Umsetzung von Benzol mit Ethylen in Gegenwart eines Zeolith-Katalysators durchgeführt wird.

5. Isoliersubstanz mit verbesserten elektrischen Isoliereigenschaften, die durch das Verfahren gemäß einem der Ansprüche 1 bis 4 erhalten wird.

## Revendications

1. Procédé d'amélioration des caractéristiques d'isolation électrique d'une fraction d'huile, qui comprend la déshydrogénation de ladite fraction à une température de l'ordre de 350 à 650°C en présence d'un catalyseur de déshydrogénation pour obtenir un mélange réactionnel déshydrogéné contenant au moins 0,5 % en poids d'une mono-oléfine aromatique ou d'une dioléfine ayant deux noyaux aromatiques condensés ou non condensés, ladite fraction à améliorer étant obtenue par les étapes de processus suivantes consistant à :
(a) faire réagir un ou plusieurs hydrocarbures aromatiques avec un agent d'alkylation en présence d'un catalyseur d'alkylation pour produire un produit d'alkylation constitué essentiellement d'hydrocarbures aromatiques mono- et polyalkylés, de diarlylalcanes, de produits plus lourds et de substances qui n'ont pas réagi.
(b) séparer la matière qui n'a pas réagi, les hydrocarbures monoaromatiques et les substances asphaltiques à partir du produit d'alkylation afin d'obtenir une fraction du produit d'alkylation ayant des points d'ébullition compris entre 255 et 420°C, et
(c) si on le souhaite, raffiner ladite fraction du produit d'alkylation.

2. Procédé selon la revendication 1, dans lequel ledit produit d'alkylation est obtenu en faisant réagir du benzène ou du toluène avec de l'éthylène, et contient principalement du benzène, de l'éthylbenzène, du polyéthylbenzène, du 1,1-diphényléthane et des produits réactionnels plus lourds.

3. Procédé selon la revendication 2, dans lequel ledit produit d'alkylation est obtenu en faisant réagir du benzène avec de l'éthylène à des températures comprises entre environ 20 et 175°C en présence d'un catalyseur de chlorure d'aluminium.

4. Procédé selon la revendication 2, dans lequel ladite réaction du benzène avec de l'éthylène s'effectue en présence d'un catalyseur de zéolite.

5. Substance d'isolation ayant des caractéristiques d'isolation électrique améliorées, obtenue par le procédé selon l'une quelconque des revendications 1 à 4.
